(19) Europäisches Patentamt
European Patent Office
Office européen des brevets

(11) EP 1 493 431 B1

(12) **EUROPÄISCHE PATENTSCHRIFT**

(45) Veröffentlichungstag und Bekanntmachung des Hinweises auf die Patenterteilung:
**30.09.2009 Patentblatt 2009/40**

(51) Int Cl.:
***A61K 8/97*** *(2006.01)* ***A61K 8/49*** *(2006.01)*
***A61K 8/41*** *(2006.01)* ***A61Q 19/00*** *(2006.01)*
***A61Q 19/08*** *(2006.01)* ***A61Q 5/00*** *(2006.01)*

(21) Anmeldenummer: **04010866.4**

(22) Anmeldetag: **07.05.2004**

(54) **Verwendung eines Extrakts aus Bauhinia zur Herstellung kosmetischer und pharmazeutischer Zubereitungen**

Use of an extract of bauhinia for the preparation of pharmaceutical and cosmetic compositions

utilisation d'un extrait de bauhinia pour la préparation de compositions pharmaceutiques ou cosmétiques

(84) Benannte Vertragsstaaten:
**AT BE BG CH CY CZ DE DK EE ES FI FR GB GR HU IE IT LI LU MC NL PL PT RO SE SI SK TR**

(30) Priorität: **03.07.2003 DE 10329955**

(43) Veröffentlichungstag der Anmeldung:
**05.01.2005 Patentblatt 2005/01**

(73) Patentinhaber: **Merck Patent GmbH**
**64293 Darmstadt (DE)**

(72) Erfinder:
- **Wirth, Corinna, Dr.**
  **64283 Darmstadt (DE)**
- **Buchholz, Herwig, Dr.**
  **60599 Frankfurt (DE)**

(56) Entgegenhaltungen:
**WO-A-02/47656**

- **DATABASE WPI Section Ch, Week 199748 Derwent Publications Ltd., London, GB; Class B04, AN 1997-521837 XP002301349 & JP 09 249523 A (POLA CHEM IND INC) 22. September 1997 (1997-09-22)**
- **DATABASE WPI Section Ch, Week 200348 Derwent Publications Ltd., London, GB; Class B04, AN 2003-508818 XP002301350 & JP 2003 055140 A (KOEI KOGYO KK) 26. Februar 2003 (2003-02-26)**
- **PATENT ABSTRACTS OF JAPAN Bd. 2003, Nr. 06, 3. Juni 2003 (2003-06-03) & JP 2003 055190 A (KOEI KOGYO KK), 26. Februar 2003 (2003-02-26)**
- **DATABASE WPI Section Ch, Week 199934 Derwent Publications Ltd., London, GB; Class B04, AN 1999-400090 XP002301351 & JP 11 158031 A (LION CORP) 15. Juni 1999 (1999-06-15)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft eine Zubereitung enthaltend einen wässrigen oder hydroalkoholischen Extrakt aus Bauhinia sowie mindestens einen weiteren hautpflegenden Inhaltsstoff ausgewählt unter Pyrimidincarbonsäuren und/oder Aryloximen, vorzugsweise Ectoin und mindestens einen für topische Anwendungen geeigneten Träger. Die erfindungsgemäße Zubereitung kann zur Pflege, Konservierung oder Verbesserung des allgemeinen Zustandes der Haut oder Haare, zur Prophylaxe oder Verhinderung von Alterungsprozessen der menschlichen Haut oder menschlicher Haare und zur Prophylaxe und/oder Behandlung von Krankheiten, die mit der Alterung der Haut zusammenhängen verwendet werden. Insbesondere kann die erfindungsgemäße Zubereitung zur Herstellung kosmetischer Zubereitungen zur Prophylaxe und/oder Verhinderung von Alterungsprozessen der Haut und zur Wundheilung verwendet werden.

Die menschliche Haut unterliegt gewissen Alterungsprozessen, die teilweise auf intrinsische Prozesse (chronoaging) und teilweise auf exogene Faktoren (environmental, z.B. photoaging) zurückzuführen sind. Zusätzlich können vorübergehende oder auch andauernde Veränderungen des Hautbildes auftreten, wie Akne, fettige oder trockene Haut, Keratosen, Rosaceae, lichtempfindliche, entzündliche, erythematöse, allergische oder autoimmunreaktive Reaktionen wie Dermatosen und Photodermatosen.

Zu den exogenen Faktoren zählen insbesondere das Sonnenlicht (UV-Licht) oder künstliche Strahlungsquellen mit vergleichbarem Spektrum sowie Verbindungen, die durch die Strahlung entstehen können, wie undefinierte reaktive Photoprodukte, die auch radikalisch oder ionisch sein können. Darüber hinaus zählen zu den exogenen Faktoren auch schädigende Umwelteinflüsse wie Luftverschmutzungen und Zigarettenrauch und die darin enthaltenen reaktiven Verbindungen wie Ozon, freie Radikale, beispielsweise das Hydroxylradikal, Singulettsauerstoff und andere reaktive Sauerstoff- oder Stickstoffverbindungen, die die natürliche Physiologie oder Morphologie der Haut stören.

Kosmetische oder dermatologische Pflegeprodukte mit Eigenschaften, die den beschriebenen oder vergleichbaren Prozessen entgegenwirken oder deren schädliche Folgen mindern oder rückgängig machen sollen, zeichnen sich häufig durch folgende spezifische Eigenschaften aus - radikalfangend, antioxidativ, entzündungshemmend oder feuchthaltend wirksam. Sie verhindern oder reduzieren u.a. die Aktivität der matrixabbauenden Enzyme oder regulieren die Neusynthese von Kollagen, Elastin oder Proteoglycanen.

Die Verwendung von Antioxidantien oder Radikalfängern in kosmetischen Zubereitungen ist an sich hinlänglich bekannt. So ist der Einsatz des antioxidativen Vitamin E in Sonnenschutzformulierungen üblich. Dennoch bleibt auch hier die erzielte Wirkung hinter der erhofften weit zurück.

Vitamin A und Vitamin-A-Derivate, wie Retinsäure, Retoinol und Retinol-Ester, wirken auf die Differenzierung von Epithelzellen und werden daher zur Prophylaxe und Behandlung zahlreicher den Hautzustand beeinträchtigender Phänomene eingesetzt, z.B. ist die Verwendung gegen Akne, Psoriaris, Altersflecken, Hautverfärbungen und Falten beschrieben. (vgl. z.B. WO 93/19743, WO 02/02074). Rentinol hat allerdings den Nachteil, dass es sehr instabil ist und außerdem erst in Retinolsäure umgewandelt werden muss, um aktiv zu sein. Retinolsäure ist überdies sehr hautreizend.

Zu den intrinsischen Vorgängen der Hautalterung, im Allgemeinen die "biologische Uhr" genannt, werden drei unterschiedliche Theorien diskutiert. Eine Theorie stützt sich auf die Beobachtung, dass diploide Zellen, wie beispielsweise Fibroblasten in Zellkultur nur ein begrenzte Lebensdauer besitzen (Hayflick-Phänomen: Campisi J. (1998), The role of cellular senescence in skin aging. J. Investig. Dermatol. Symp. Proc. 3:1-5). Die Folge ist eine zelluläre Seneszenz, die zu veränderter Genexpression und zu degenerativen Veränderungen im Gewebe führt.

Eine weiterer intrinsischer Mechanismus, der zur Hautalterung beiträgt, ist die Akkumulation freier Radikale in den Zellen über die Lebensdauer eines Individuums hinweg und die Zellschädigung durch diese freien Radikale (Biesalski H. K. (2002), Free radical theory of aging, Curr. Opin. Clin. Nutr. Metab. Care, Band 5).

Die Glykosylierungstheorie (Maillard-Theorie: Kasper, M. & Funk, R. H. W. (2001), Age-related changes in cells and tissues due to advanced glycation end products (AGEs), Archives of Gerontology and Geriatrics 32: 233-243; Reiser, K. M. (1998), Nonenzymatic glycation of collagen in aging & diabetes, Proc. Soc. Exp. Biol. Med. 218: 23-37) ist heute allgemein als weiterer intrinsischer Alterungsmechanismus anerkannt (Glykosylierung, Glykosidierung oder Glycation von Proteinen). Nichtenzymatische, spontane chemische Reaktionen zwischen Proteinen und Zuckern führen zu veränderter Struktur und Funktionsweise von Gewebeproteinen (Proteine der extrazellulären Matrix, z.B. Kollagen) und zu Endprodukten fortgeschrittener Glykosylierung, sogenannten "AGEs" (advanced glycation end products). Über Matrix-Zell-Wechselwirkungen werden Zelleigenschaften wie Migration, Wachstum, Differenzierung, Proliferation und Genexpression und damit letztendlich auch die Gewebestruktur durch die veränderten Proteine der extrazellulären Matrix oder AGEs beeinflusst.

Durch den Einfluss vorgenannter exogener und intrinsischer Faktoren kann es unter anderem zu direkten Schäden an der DNA der Hautzellen kommen sowie an den Kollagen-, Elastin- oder Glycosaminoglycanmolekülen der extrazellulären Matrix, die für die Festigkeit der Haut verantwortlich sind. Darüber hinaus kann es zu einer Beeinflussung der Signaltransduktionsketten kommen, an deren Ende die Aktivierung matrixabbauender Enzyme steht. Wichtige Vertreter dieser Enzyme sind die Matrixmetalloproteinasen (MMPs, z.B. Kollagenasen, Gelatinasen, Stromelysine), deren Aktivität zusätzlich durch TIMPs (tissue inhibitor of matrix metalloproteinases) reguliert wird.

Die Folgen der o.g. Alterungsprozesse sind Verdünnung der Haut, schwächere Verzahnung von Epidermis und Dermis, Reduktion der Zellzahl sowie der versorgenden Blutgefäße. Dabei kommt es zur Ausbildung von feinen Linien und Falten, die Haut wird ledrig und es können Pigmentstörungen auftreten.

Die gleichen Faktoren wirken auch auf die Haare, wo es ebenfalls zu einer Schädigung kommen kann. Die Haare werden spröde, weniger elastisch und glanzlos. Die Oberflächenstruktur der Haare wird geschädigt.

Zur Hemmung der obengenannten AGE-Bildung bei der Protein-Glykosylierung gibt es verschiedene denkbare Angriffspunkte. Inhibitoren können als Zucker-Kompetitoren wirken oder freie Aminogruppen von Proteinen modifizieren, um eine Anheftung von Zuckern zu verhindern. Ein Beispiel solcher Inhibitoren ist Aspirin, das die Glykosylierung blockiert, indem es Lysin-Reste acetyliert. Andere Inhibitoren reagieren mit Aldose- oder Ketose-Zuckern (Protein-Kompetitoren) und verhindern so Maillard-Reaktionen mit Proteinen (AGE-Bildung). Diese Inhibitorengruppe umfasst Verbindungen mit freien Aminogruppen, wie beispielsweise die Aminosäurereste Lysin und Arginin, sowie Verbindungen wie Carnosin oder Ethanolamin. Der wirksamste bekannte Inhibitor, Aminoguanidin, greift möglicherweise in zwei Schritte der Maillard-Kaskade ein (Khalifah, R. G. et al. (1999), Amadorins: novel post-Amadori inhibitors of advanced glycation reactions, Biochem. Biophys. Res. Commun. 257: 251-258). So reagiert Aminoguanidin mit Amadori-Verbindungen, die Hemmung wird jedoch vermutlich durch Einschluss reaktiver Dicarbonyl-Intermediate vermittelt, die aus Oxidationsreaktionen von freien Zuckern oder Amadori-Produkten hervorgehen. Aminoguanidin weist jedoch als Hydrazinmedikament ungünstige Nebenwirkungen auf, da es dem Körper essentielle Carbonylverbindungen wie beispielsweise Pyridoxal-5'-phosphat (Vitamin B6) entzieht.

Weitere Inhibitoren wie Pyridoxamin oder Thiaminpyrophosphat gelten als post-Amadori-Inhibitoren, da sie am wirkungsvollsten die Umwandlung von Amadori-Intermediaten in AGEs hemmen (Khalifah, R. G. et al. (1999), Amadorins: novel post-Amadori inhibitors of advanced glycation reactions, Biochem. Biophys. Res. Commun. 257: 251-258). Da die AGE-Bildung von Oxidationsreaktionen abhängig ist, ist die Verwendung von Antioxidantien wie Vitamin C und E oder des Pflanzencytokins Kinetin ein weiterer Ansatz zur Verhinderung fortschreitender Glykosylierung (Verbeke P. et al. (2000), Kinetin inhibits protein oxidation and glycoxidation in vitro. Biochem. Biophys. Res. Commun. 276: 1265-1270.)

Die pharmazeutische Verwendung von Aminoguanidin, Pyridoxamin und Aspirin eignet sich zwar beispielsweise für die Behandlung von Diabetes, bisher bekannte Wirkstoffe eignen sich jedoch nur begrenzt für topische Anwendungen. Wirkstoffe für topische Anwendungen kosmetischer Produkte sollen ausreichend oxidationsund photostabil sowie gut formulierbar sein. Zubereitungen, die solche Wirkstoffe oder Wirkstoffkombinationen enthalten, sollen ferner sehr gut verträglich sein, keine Toxizität, keine Nebenwirkungen und möglichst ein niedriges Irritationspotential für die Haut aufweisen. Außerdem sollen sie möglichst die Wasserbindung in der Haut positiv beeinflussen und die Elastizität der Haut erhalten oder erhöhen und somit eine Glättung der Haut fördern. Darüber hinaus sollen sie vorzugsweise beim Auftragen auf die Haut ein angenehmes Hautgefühl erzeugen.

Aufgrund des immer größer werdenden Bedarfs an Wirkstoffen zur vorbeugenden Behandlung menschlicher Haut und menschlicher Haare gegen Alterungsprozesse und schädigende Umwelteinflüsse, war es Aufgabe der vorliegenden Erfindung, neue Wirkstoffe oder Wirkstoffkombinationen bereitzustellen, die die Glykosylierung von Proteinen und damit Alterungsprozesse im Bereich der Haut und der Haare wirksam verhindern und sich darüber hinaus für die topische Anwendung eignen.

Überraschenderweise wurde gefunden, dass eine Zubereitung enthaltend einen wässrigen oder hydroalkoholischen Extrakt aus Bauhinia sowie mindestens einen weiteren hautpflegenden Inhaltsstoff ausgewählt unter Pyrimidincarbonsäuren und/oder Aryloximen, vorzugsweise Ectoin und mindestens einen für topische Anwendungen geeigneten Träger eine außergewöhnlich gute, die Protein-Glykosylierung hemmende Wirkung zeigt und zur Behandlung und Prophylaxe von Alterungsprozessen der menschliche Haut oder menschlicher Haare verwendet werden kann. Die erfindungsgemäßen Zubereitungen sind sehr gut verträglich und weisen keine Toxizität, Nebenwirkungen oder Irritationspotential auf.

Gegenstand der Erfindung ist deshalb eine Zubereitung enthaltend einen wässrigen oder hydroalkoholischen Extrakt aus Bauhinia sowie mindestens einen weiteren hautpflegenden Inhaltsstoff ausgewählt unter Pyrimidincarbonsäuren und/oder Aryloximen, vorzugsweise Ectoin und mindestens einen für topische Anwendungen geeigneten Träger. Die erfindungsgemäßen Zubereitungen werden vorzugsweise zur Pflege, Konservierung oder Verbesserung des allgemeinen Zustandes der Haut oder der Haare verwendet.

Insbesondere können die erfindungsgemäßen Zubereitungen zur Prophylaxe und/oder Verhinderung von Alterungsprozessen der menschlichen Haut oder menschlicher Haare, insbesondere zur Prophylaxe, Verhinderung und/oder Reduktion vorzeitiger Hautalterung, von trockener Haut, Hautunebenheiten, wie Falten, feinen Linien, rauer Haut oder großporiger Haut und/oder Pigmentstörungen verwendet werden.

Außerdem eignen sich die erfindungsgemäßen Zubereitungen zur kosmetichen Prophylaxe und/oder Verhinderung von Alterungsprozessen der menschlichen Haut oder menschlicher Haare, die mit der Glykosylierung von Proteinen zusammenhängen.

Insbesondere eignen sich erfindungsgemäße Zubereitungen auch kosmetische zur Behebung oder Bekämpfung der lichtbedingten und der mit dem Älterwerden zusammenhängenden Hautalterung, zur Verminderung der Pigmentierungen zur Vermeidung oder Behebung von Schwangerschaftsstreifen zur Bekämpfung von Störungen der Talgproduktion, wie der einfachen Seborrhö, , zur Vorbeugung vor oder Behandlung der Alopecie, und zur kosmetische Behandlung von Hautproblemen, die durch UV-Strahlung hervorgerufen werden.

Die Glykosylierungstheorie der Alterung wurde erstmals von A. Cerami (Cerami A. (1985), Hypothesis: Glucose as a Mediator of Aging, Journal of the American Geriatric Society, 33(9) pages 626-634) und Monnier (Hayase F. et al. (1989), Aging of proteins: immunological detection of a glucose-derived pyrrole formed during maillard reaction in vivo, J. Biol. Chem. Mar. 5; 264 (7):3758-64) vorgestellt. Kollagen und Elastin, die zwei wichtigsten Strukturproteine menschlichen Gewebes, sind auch im physiologischen Zustand Gegenstand molekularer Veränderungen wie Crosslinkings und Seitenkettenmodifikationen. Pyridinolin-Crosslinkings werden enzymatisch durch die Lysyloxidase ausgeführt. Dieser präzise enzymatische Vorgang ist für die normale Entwicklung der extrazellulären Matrix essentiell. Nichtenzymatisch gebildete Crosslinkings und Polymerisationen dagegen sind das Ergebnis spontaner Reaktionen zwischen Proteinen und Zuckern. Glykosylierende Agenzien sind hier neben Glukose und anderen Zuckern auch Oxoaldehyde, Glyoxal, Methylglyoxal und 3-Deoxyglucoson. Neben Proteinen können auch Nukleotide und Lipide spontan glykosyliert werden. Glukose in Aldehydform reagiert mit einer Aminogruppe eines Proteins unter Bildung einer Schiff'schen Base die in ein stabiles Ketoamid (Fructosamin) umgelagert wird (Amadori-Umlagerungen). Diese frühen Glykosylierungsprodukte werden über kovalente und irreversible Crosslinkings vernetzt und polymerisiert (Maillard-Reaktionen), so dass braungefärbte Glykosylierungsendprodukte (AGEs) entstehen, im Fall von Proteinen der extrazellulären Matrix zu Proteinen mit veränderten chemischen und biologischen Eigenschaften (abnorme strukturelle Stabilität oder Denaturierung der Proteine). Die AGE-Strukturen, Pentosidin und Mold (Methylglyoxal-Lysin-Dimer), sind Crosslinks zwischen Lysin und Arginin bzw. zwischen zwei Lysin-Resten. Insbesondere sind Proteine mit langer Lebensdauer wie beispielsweise Kollagen betroffen, so dass chemische Schädigungen mit zunehmendem Alter akkumulieren.

Die beschriebene Protein-Glykosylierung hat verschiedene negative Auswirkungen auf die Eigenschaften des Kollagens. So konnte gezeigt werden (Verzijl N. et al. (2000 Sep 1), Age-related accumulation of Maillard reaction products in human articular cartilage collagen Biochem. J.; 350 Pt 2:381-7), dass die Akkumulation von Maillard-Produkten zu steiferem und brüchigerem Kollagen führt. Außerdem beeinflusst die Glykosylierung die korrekte Zusammenlagerung von Kollagen-Monomeren zu Fasern und bei Diabetes-Patienten wurden signifikanten Veränderungen bezüglich der AGEs im Kollagen der Haut gefunden (Beisswenger, P. J. et al. (1993) Diabetes Care 16: 689-694). Glykosylierung verändert nicht nur die Eigenschaften des Kollagens und der extrazellulären Matrix, sondern modifiziert auch die Matrix-Zell-Wechselwirkungen. Da die extrazelluläre Matrix zahlreiche Eigenschaften der benachbarten Zellen bestimmt, einschließlich Migration, Wachstum, Differenzierung und Genexpression, können diese durch Veränderungen der Matrix-Komponenten, wie beispielsweise die nichtenzymatische Glykosylierung von Kollagen, nachhaltig beeinflusst werden.

So konnte gezeigt werden, dass sich Zellen, die auf einer Matrix aus glykosylierten Proteinen kultiviert werden, im Allgemeinen bezüglich ihres Wachstums, ihrer Differenzierung, Motilität, Genexpression und Antwort auf Cytokine von solchen Zellen unterscheiden, die auf normaler Matrix kultiviert werden. Es wurden verschiedene Rezeptoren für AGEs (RAGE; OST-48, 80K-H, Galectin-3, Scavenger-Rezeptor) gefunden, die auf einer Vielzahl unterschiedlicher Zellen exprimiert werden. Über die AGE-Rezeptoren werden veränderte Zelleigenschaften vermittelt. So kommt es vermutlich zu Zellaktivierung und zu Fehlfunktionen, aber auch das Abwehrsystem des Körpers gegen AGEs wird vermutlich über diese Rezeptoren gelenkt, beispielsweise Makrophagen beseitigen veränderte Proteine. Alterungsprozessen liegt wohl unter anderen auch ein Verlust dieses Abwehrmechanismus zugrunde.

Die vorgenannten Prozesse, wie Glykosylierung von Proteinen (sowie anderer Moleküle), Crosslinking und Bildung von AGEs und damit Veränderungen der extrazellulären Matrix (Beeinträchtigung der Struktur und Funktion von Gewebeproteinen) und Matrix-Zell-Wechselwirkungen, führen schließlich zu Zell-, Gewebe- und Organveränderungen, wie sie bei Alterungsprozessen oder auch in ähnlicher Weise bei der Pathophysiologie infolge von Diabetes auftreten.

Überraschenderweise können erfindungsgemäße Zubereitungen die Protein-Glykosylierung und Alterungsprozesse im Bereich der Haut und der Haare wirksam verhindern.

Der zur Herstellung einer erfindungsgemäßen Zubereitung verwendete wässrige oder hydroalkoholische Extrakt kann aus allen bekannten Bauhinia-Arten erhalten werden.

Gegenstand der Erfindung ist deshalb die erfindungsgemäße Verwendung eines wässrigen oder hydroalkoholischen Bauhinia-Extrakts zur Herstellung einer Zubereitung, **dadurch gekennzeichnet, dass** der Bauhinia-Extrakt aus einer Bauhinia-Species erhalten wird ausgewählt aus Bauhinia candicans, Bauhinia championii, Bauhinia fortificata (= Bauhinia forficata), Bauhinia manca, Bauhinia purpurea, Bauhinia racemosa, Bauhinia reticulata, Bauhinia tomentosa, Bauhinia variegata, Bauhinia cheilantha, Bauhinia guanensis, Bauhinia refusa, Bauhinia glauca, Bauhinia pauletia, Bauhinia ungulato, Bauhinia macrostachya und Bauhinia splendens. Bevorzugt ist der Extrakt aus Bauhinia variegata. Besonders bevorzugt ist der aus Bauhinia fortificata (= Bauhinia forficata) gewonnene Extrakt.

Ein bevorzugter Gegenstand der Erfindung sind erfindungsgemäße Zubereitungen, **dadurch gekennzeichnet, dass** die Zubereitung einen wässrigen oder hydroalkoholischen Extrakt aus Bauhinia in einer Menge von 0,001 bis 20 Gew.-%, vorzugsweise in einer Menge von 0,01 bis 10 Gew.-% enthält. Besonders bevorzugt enthält die Zubereitung

einen wässrigen oder hydroalkoholischen Extrakt aus Bauhinia in einer Menge von 0,1 bis 8 Gew.-%. Dabei bereitet es dem Fachmann keinerlei Schwierigkeiten die Mengen abhängig von der beabsichtigten Wirkung der Zubereitung entsprechend auszuwählen.

Im Prinzip können alle äußeren Pflanzenteile zur Extraktgewinnung verwendet werden, die besten Ergebnisse werden mit Extrakten aus Blättern, insbesondere jungen Blättern von Bauhinia erhalten. Deshalb ist die Verwendung von Extrakten aus jungen Blättern ein besonders bevorzugter Gegenstand der vorliegenden Erfindung.

Unsere Untersuchungen zeigen, dass sich die hohe Wirksamkeit der Bauhinia-Extrakte aus einer Kombination der verschiedenen Inhaltsstoffe des Extrakts ergibt.

In den Extrakten wurden nachfolgende Bestandteile oder Inhaltsstoffe identifiziert:

- aus Bauhinia candicans: Rutin, Quercetin, Quercitrin, Isoquercetin (Isoquercitrin), Campesterol, Stigmasterol, Cholesterol, Stigmast-3,5-dien-7-on, Triacontanol, Cholin, Trigonellin, Trigonellin-Acetate, Kaempferol-3-rutinosid, Kaempferol-3-rutinosid-7-rhamnosid, Sitosterol-3-glycosid (äußere Teile), Sitosterol-3-O-β-D-xylopyranosid, Sitosterol-3-O-α-D-ribunonosofuranosid, 3-O-Methyl-D-inositol (D-Pinit), Sitosterol-3-O-D-xyluronofuranosid;

- aus Bauhinia championii: 5,6,7,5'-Tetramethylendioxy-3',4',methylendioxyflavon, 5,6,7,5,3',4',5'-Hexamethoxyflavon,5,7,5'-trimethoxy-3',4',methylendioxyflavon;

- aus Bauhinia fortificata: Quercetin, Quercetin-3,7-O-α-dirhamnosid, Isoquercetin (Isoquercitrin), Kaempferol-3-rutinosid, Rutin, Quercitrin, Campesterol, Stigmasterol, Cholesterol, Stigmast-3,5-dien-7-on, Triacontanol, Cholin, Trigonellin, Kaempferol, Kaempferol-7-O-α-rhamnosid, Kaempferol-3-rutinosid-7-rhamnosid, Kaempferol 3,7-dirhamnosid (Kaempferitrin), Sitosterol-3-glycosid (äußere Teile), 3-O-Methyl-D-inositol (D-Pinit), Beta-sitosterol, Daucosterol, Lupeol, Saponine, Tannine, Astragalin;

- aus Bauhinia manca: p-Cumarinsäure, Ferulinsäure, Phytosterole, Zimtsäure, Gallsäure, Epicatechin-3-gallat, 5,7-Ihydroxychromon, Hydroxypropioguaiacon, Obtustyren, Isoliquitigenin-4-methylether, Liquiritigenin-4'-methylether, 2,4'-Dihydroxy-4-methoxydihydrochalcon, 4'-Hydroxy-7,3'-dimethoxyflavan, 3',4'-Dihydroxy-7-methoxyflavan, Syringaresinol, 5,5'-dimethoxylariciresinol, Chrysoeriol, Luteolin-5,3'-dimethylether;

- aus Bauhinia purpurea: 6'-(Stigmast-5-en-7-on-3-O-glucopyranosidyl)-hexadecanoat, 3-Hydroxystigmast-5-en-7-on, Oleanolsäure, 6,8-Dimethylchrysin, Chrysin, Isoquercetin (Isoquercitrin), Astragalin, 2,3-Dihydroxypropyl-oleat, 2,3-Dihydroxypropyllinoleat, 2,3-Dihydroxypropyl-16-hydroxyhexadecanoat, 6-Butyl-3-hydroxyflavavon, (6-(3''-Oxobutyl)-taxifolin, 5,6-Dihydroxy-7-methoxyflavon, 6-O-D-Xylopyrynose;

- aus Bauhinia racemosa: Kaempferol, Quercetin, Kaempferol-3-O-rhamnosid, Quercitrin, D-O-Methylracemosol , Pacharin;

- aus Bauhinia reticulata: Quercetin, Quercitrin;

- aus Bauhinia tomentosa: Rutin, Quercetin, Isoquercetin (Isoquercitrin);

- aus Bauhinia variegata: Apigenin, Apigenin-7-O-glucosid, Kaempferol-3-galactosid, Kaempferol-3-rhamno-glycosid, Kaempferol-3-glucosid (Astragalin), Sitosterol, Lupeol, Naringenin-4'-rhamnoglucosid, Naringin 5,7-dimethyl-ether-4'rhamnoglycosid, 5,7-Dihydroxyflavonanon-4'-O-L-rhamnoyranosyl-β-Dglucopyranosid (Naringeninaglycon), aus Hydrolyseergebnissen: Quercetin in Blättern, Blüten, Samen, Myricetin in Samen, Dihydroxyquercetin (Taxifolin) im Perikarp, Kaempferol in Blüten, Methylester von Kaempferol in Blüten, Methylesters von Quercetin in Blättern, Taxifolin, Rutin, Quercetin, Quercitrin, Isoquercetin (Isoquercitrin);

- aus Bauhinia guanensis (in der Stammrinde): Beta-sitosterol, Stigmasterol, 3-O-Glucopyranosylstigmasta-5-22-dien, 3-O-Glucopyranosyl-sitostreol, 4'-Hydroxy-7-methoxyflavan, Lapachol.

Die vorliegenden Ergebnisse zeigen, dass erfindungsgemäße Extrakte die Protein-Glykosylierung außerordentlich wirksam hemmen. Die Wirksamkeit ist der reiner Wirkstoffe wie beispielsweise Aminoguanidin, Aspirin oder Carnosin vergleichbar.

Man nimmt an, dass sich die hohe Wirksamkeit der Extrakte aus der Kombination wenigstens zweier Wirkstoffe ergibt. Vermutlich ist wenigstens einer dieser Wirkstoffe ein Flavon oder Flavonoid, wie Apigenin, Apigenin-7-O-glucosid, Isoquercetin (Isoquercitrin), Kaempferol-3-rutinosid, Kaempferol-3-galactosid, Kaempferol-3-rhamno-glycosid, Kaempferol-3-glucosid (Astragalin), Naringenin-4'rhamnoglucosid, Naringin, Quercetin, Quercetin-3,7-O-α-dirhamnosid, Quercitrin, 5,7-Dimethyl-ether-4'-rhamnoglycosid, Rutin, 5,7-Dihydroxyflavanon-4'-O-Lrhamnopyranosyl-β-D-glucopyranosid (Naringeninaglycon), vorzugsweise ist wenigstens ein Wirkstoff ausgewählt aus Quercetin, Isoquercetin or Rutin.

Weitere Inhaltsstoffe erfindungsgemäßer Bauhinia-Extrakte umfassen Polypeptide, Polysaccharide, Steroide and Saponine. In einer bevorzugten Ausführungsform der vorliegenden Erfindung enthält der verwendete Extrakt wenigstens einen der vorgenannten Wirkstoffe.

Erfindungsgemäße Bauhinia-Extrakte umfassen alle Extrakte aus obengenannten Bauhinia-Species, die nach einem dem Fachmann bekannten Extraktionsverfahren hergestellt wurden. Insbesondere sind auch Bauhinia-Extrakte Gegenstand der Erfindung, die nach einem dem Fachmann bekannten Verfahren weiter aufgereinigt wurden. Erfindungsgemäß sind weiterhin auch Fraktionen der genannten Extrakte bis hin zu isolierten Einzelverbindungen, die aus den aus den erfindungsgemäßen Bauhinia-Species selbst oder aus deren Extrakten isoliert wurden, sowie synthetisch hergestellte Verbindungen, die in natürlicher Form in wenigstens einer der obengenannten Bauhinia-Species enthalten sind.

Unabhängig von den genannten analytischen Ergebnissen wurde gefunden, dass insbesondere Extrakte, die durch Extraktion mithilfe eines Gemischs polarer Lösungsmittel und anschließendem Verwerfen des Lösungsmittels aus Teilen von Bauhinia erhalten wurden, ausgezeichnete Ergebnisse zeigen.

Bevorzugter Gegenstand der Erfindung sind deshalb erfindungsgemäße Zubereitungen, **dadurch gekennzeichnet, dass** der Bauhinia-Extrakt erhalten wird durch

a) Extraktion von Teilen von Bauhinia mithilfe eines Gemischs polarer Lösungsmittel und
b) anschließendes Verwerfen den Lösungsmittels.

Die Mischung der polaren Lösungsmittel umfasst vorzugsweise Wasser und wenigstens ein weiteres Lösungsmittel, ausgewählt aus Lösungsmitteln, die weniger polar sind als Wasser. Eine bevorzugte Auswahl solcher Lösungsmittel umfasst Methanol, Ethanol, 1-Propanol, 2-Propanol, Acetonitril, Aceton and Ethylacetat. Ein bevorzugtes Lösungsmittelgemisch enthält 10 - 90 % (Vol) Wasser, besonders bevorzugt enthält das Lösungsmittelgemisch 30 - 70 % (Vol) Wasser.

Die Extraktion kann beispielsweise wie folgt ausgeführt werden:

Die wirksamen Bestandteile werden aus getrockneten Bauhinia-Blättern mithilfe eines Gemischs aus Ethanol und Wasser extrahiert, mit einem Ethanolgehalt von 50% (Vol). Die Extraktion wird bei einer Temperatur zwischen 20 und 90°C durchgeführt, vorzugsweise zwischen 50 und 90°C, besonders bevorzugt bei 70°C. Nach dem Abfiltrieren des Lösungsmittels kann der Vorgang zur Erhöhung der Ausbeute wiederholt werden. Der gewonnene Extrakt liegt als Konzentrat vor. Das Konzentrat wird dann getrocknet, vorzugsweise durch Sprühtrocknung. Geeignete Bedingungen zur Sprühtrocknung sind in Beispiel 1 beschrieben.

Ein weiterer bevorzugter Gegenstand der Erfindung sind erfindungsgemäße Zubereitungen, **dadurch gekennzeichnet, dass** der Bauhinia-Extrakt erhalten wird durch wässrige Extraktion aus Teilen von Bauhinia. Dabei erfolgt die Extraktion nach oben angegebenem Verfahren, wobei anstelle des obengenannten Gemischs polarer Lösungsmittel (hydroalkoholisches Gemisch) Wasser verwendet wird.

Alle Verbindungen oder Komponenten, die in den Zubereitungen verwendet werden können, sind entweder bekannt und käuflich erwerbbar oder können nach bekannten Verfahren synthetisiert werden. Bei den hautschonenden oder hautpflegenden Wirkstoffen kann es sich prinzipiell um alle den Fachmann bekannten Wirkstoffe handeln.

Die erfindungsgemäßen Zubereitungen enthalten mindestens einen weiteren hautpflegenden Inhaltsstoff ausgewählt unter Pyrimidincarbonsäuren und/oder Aryloxime, vorzugsweise Ectoin und mindestens einen für topische Anwendungen geeigneten Träger.

Nachfolgend werden weitere verwendbare hautpflegende Inhaltsstoffe und Träger näher beschrieben. Hautpflegende Inhaltsstoffe sind insbesondere Antioxidantien und/oder Vitamine, insbesondere ausgewählt unter Vitamin C und dessen Derivaten, DL-α-Tocopherol, Tocopherol-E-Acetat, Nicotinsäure, Pantothensäure, Biotin, Flavonoiden, Glutathion, Coenzym Q10, protein-gebundenem Zink oder Selen und α-Liponsäure.

Wie oben beschrieben, können oxidativer Stress oder die Schädigung durch freie Radikale die verschiedenen Alterungsprozesse verursachen oder beeinflussen. In der Literatur wird beschrieben, dass auch die AGE-Bildung von Oxidationsprozessen abhängig sein kann. Ein weiterer Vorteil eines erfindungsgemäßen Extrakts aus Bauhinia ist deshalb dessen antioxidative Wirkung, die vermutlich durch die enthaltenen Flavonoide vermittelt wird. Die schützende Wirkung erfindungsgemäßer Zubereitungen vor oxidativen Einflüssen bzw. gegen die Einwirkung von Radikalen kann aber auch weiter verbessert werden, wenn die Zubereitungen ein oder mehrere weitere Antioxidantien enthalten, wobei es dem Fachmann keinerlei Schwierigkeiten bereitet geeignet schnell oder zeitverzögert wirkende Antioxidantien auszuwählen.

Es gibt viele aus der Fachliteratur bekannte und bewährte Substanzen, die als Antioxidantien verwendet werden

können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, $ZnSO_4$), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT); L-(+)-Ascorbylpalmitat und Zitronensäure (z.B. Oxynex® 2004). Derartige Antioxidantien werden mit dem Bauhinioa-Extrakt in solchen Zusammensetzungen üblicherweise in Verhältnissen im Bereich von 1000:1 bis 1:1000, bevorzugt in Mengen von 100:1 bis 1:100 eingesetzt.

Die erfindungsgemäßen Zubereitungen können als weitere Inhaltsstoffe Vitamine enthalten. Bevorzugt sind Vitamine und Vitamin-Derivate ausgewählt aus Vitamin B, Thiaminchloridhydrochlorid (Vitamin $B_1$), Riboflavin (Vitamin $B_2$), Nicotinsäureamid, Vitamin C (Ascorbinsäure), Vitamin D, Ergocalciferol (Vitamin $D_2$), Vitamin E, DL-α-Tocopherol, Tocopherol-E-Acetat, Tocopherolhydrogensuccinat, Vitamin $K_1$, Esculin (Vitamin P-Wirkstoff), Thiamin (Vitamin $B_1$), Nicotinsäure (Niacin), Pyridoxin, Pyridoxal, Pyridoxamin, (Vitamin $B_6$), Panthothensäure, Biotin, Folsäure und Cobalamin (Vitamin $B_{12}$) in den erfindungsgemäßen kosmetischen Zubereitungen enthalten, insbesondere bevorzugt Vitamin C und dessen Derivaten, DL-α-Tocopherol, Tocopherol-E-Acetat, Nicotinsäure, Pantothensäure und Biotin. Vitamine werden dabei mit dem Bauhinia-Extrakt üblicherweise in Verhältnissen im Bereich von 1000:1 bis 1:1000, bevorzugt in Mengen von 100:1 bis 1:100 eingesetzt.

Unter den Phenolen mit antioxidativer Wirkung sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydoxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es wird dort beobachtet, dass Dihydroxyflavone mit einer OH-Gruppe benachbart zur Ketofunktion oder OH-Gruppen Gruppen in 3'4'- oder 6,7- oder 7,8-Position antioxidative Eigenschaften aufweisen, während andere Mono- und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers, I.M.C.M. Rietjens; Free Radical Biology&Medicine 2001, 31(7), 869-881 untersuchen die pH-Abhängigkeit der antioxidanten Wirkung von Hydoxyflavonen. Über den gesamten pH-Bereich zeigt Quercetin die höchste Aktivität der untersuchten Strukturen.

Die erfindungsgemäßen Zubereitungen enthalten mindestens einen weiteren hautpflegenden Inhaltsstoff_ausgewählt unter Pyrimidincarbonsäuren und/oder Aryloxime, vorzugsweise Ectoin und mindestens einen für topische Anwendungen geeigneten Träger.

Pyrimidincarbonsäuren kommen in halophilen Mikroorganismen vor und spielen bei der Osmoregulation dieser Organismen eine Rolle (E.A. Galinski et al., Eur. J. Biochem., 149 (1985) Seite 135-139). Dabei sind unter den Pyrimidincarbonsäuren insbesondere Ectoin ((S)-1,4,5,6-Tetrahydro-2-methyl-4-pyrimidincarbonsäure) und Hydroxyectoin ((S, S)-1,4,5,6-Tetrahydro-5-hydroxy-2-methyl-4-pyrimidincarbonsäure und deren Derivate zu nennen. Diese Verbindungen stabilisieren Enzyme und andere Biomoleküle in wässrigen Lösungen und organischen Lösungsmitteln. Weiter stabilisieren sie insbesondere Enzyme gegen denaturierende Bedingungen, wie Salze, extreme pH-Werte, Tenside, Harnstoff,

Guanidiniumchlorid und andere Verbindungen.

Ectoin und Ectoin-Derivate wie Hydroxyectoin können vorteilhaft in Arzneimitteln verwendet werden. Insbesondere kann Hydroxyectoin zur Herstellung eines Arzneimittels zur Behandlung von Hauterkrankungen eingesetzt werden. Andere Einsatzgebiete des Hydroxyectoins und anderer Ectoin-Derivate liegen typischerweise in Gebieten in denen z.B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutische wirksame Peptide und Proteine z.B. t-PA können mit Ectoin oder seinen Derivaten geschützt werden.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Ups, Pflegecremes und Sonnenschutzpräparaten eingesetzt werden.

Dabei wird vorzugsweise eine Pyrimidincarbonsäure gemäß der unten stehenden Formel II eingesetzt,

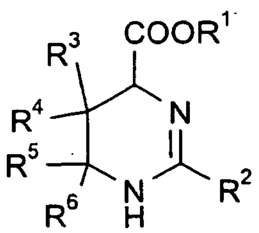

II

worin $R^1$ ein Rest H oder C1-8-Alkyl, $R^2$ ein Rest H oder C1-4-Alkyl und $R^3$, $R^4$, $R^5$ sowie $R^6$ jeweils unabhängig voneinander ein Rest aus der Gruppe H, OH, $NH_2$ und C1-4-Alkyl sind. Bevorzugt werden Pyrimidincarbonsäuren eingesetzt, bei denen $R^2$ eine Methyl- oder eine Ethylgruppe ist und $R^1$ bzw. $R^5$ und $R^6$ H sind. Insbesondere bevorzugt werden die Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidin-carbonsäure) eingesetzt. Dabei enthalten die erfindungsgemäßen Zubereitungen derartige Pyrimidincarbonsäuren vorzugsweise in Mengen bis zu 15 Gew.-%. Vorzugsweise werden die Pyrimidincarbonsäuren dabei in Verhältnissen von 100:1 bis 1:100 zum Bauhinia-Extrakt eingesetzt, wobei Verhältnisse im Bereich 1:10 bis 10:1 besonders bevorzugt sind.

Unter den Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE-A-41 16 123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Erfindungsgemäße Zubereitungen, die neben de Bauhinia-Extrakt zusätzlich ein Aryloxim, vorzugsweise 2-Hydroxy-5-methyllaurophenonoxim enthalten, zeigen überraschende antiinflammatorische Eignung. Dabei enthalten die Zubereitungen vorzugsweise 0,01 bis 10 Gew.-% des Aryloxims, wobei es insbesondere bevorzugt ist, wenn die Zubereitung 0,05 bis 5 Gew.-% Aryloxim enthält.

Ein weiterer Gegenstand der vorliegenden Erfindung sind Zubereitungen, enthaltend mindestens einen weiteren Wirkstoff, ausgewählt unter Tilirosid, Aspirin, Lysin, Arginin, Carnosin, Ethanolamin, Aminoguanidin, Pyridoxamin, Thiaminpyrophosphat, Kinetin, Vitamin C und Vitamin E.

Bevorzugte erfindungsgemäße Zubereitungen enthalten außerdem UV-Filter.

Gegenstand der Erfindung sind deshalb Zubereitungen, enthaltend einen oder mehrere UV-Filter, ausgewählt unter 3-(4'-Methylbenzyliden)-dl-kampfer, 1-(4-tert-Butylphenyl)-3-(4-methoxyphenyl)propan-1,3-dion, 4-Isopropyldibenzoylmethan, 2-Hydroxy-4-methoxybenzophenon, Methoxyzimtsäureoctylester, 3,3,5-Trimethylcyclohexylsalicylat, 4-(Dimethylamino)benzoesäure-2-ethylhexylester, 2-Cyano-3,3-diphenylacrylsäure-2-ethylhexylester und 2-Phenylbenzimidazol-5-sulfonsäure sowie ihren Kalium-, Natrium- und Triethanolaminsalzen, anorganischen Filtern und verkapselten Filtern.

Gegenstand der vorliegenden Erfindung ist auch erfindungsgemäße Zubereitungen, **dadurch gekennzeichnet, dass** sie einen oder mehrere UV-Filter enthalten, ausgewählt unter 3-(4'-Methylbenzyliden)-dl-kampfer, 1-(4-tert-Butylphenyl)-3-(4-methoxyphenyl)propan-1,3-dion, 4-Isopropyldibenzoylmethan, 2-Hydroxy-4-methoxybenzophenon, Methoxyzimtsäureoctylester, 3,3,5-Trimethylcyclohexylsalicylat, 4-(Dimethylamino)benzoesäure-2-ethylhexylester, 2-Cyano-3,3-diphenylacrylsäure-2-ethylhexylester und 2-Phenylbenzimidazol-5-sulfonsäure sowie ihren Kalium-, Natrium- und Triethanolaminsalzen, anorganischen Filtern und verkapselten Filtern.

Prinzipiell kommen alle UV-Filter für eine Kombination mit dem erfindungsgemäßen Bauhinia-Extrakt in Frage. Besonders bevorzugt sind solche UV-Filter, deren physiologische Unbedenklichkeit bereits nachgewiesen ist. Sowohl für UVA wie auch UVB-Filter gibt es viele aus der Fachliteratur bekannte und bewährte Substanzen, z.B.:

Benzylidenkampferderivate wie 3-(4'-Methylbenzyliden)-dl-kampfer (z.B. Eusolex® 6300), 3-Benzylidenkampfer (z.B. Mexoryl® SD), Polymere von N-{(2 und 4)-[(2-oxoborn-3-yliden)methyl]benzyl}-acrylamid (z.B. Mexoryl® SW), N,N,N-Trimethyl-4-(2-oxoborn-3-ylidenmethyl)anilinium methylsulfat (z.B. Mexoryl® SK) oder (2-Oxoborn-3-yliden) toluol-4-sulfonsäure (z.B. Mexoryl® SL),

Benzoyl- oder Dibenzoylmethane wie 1-(4-tert-Butylphenyl)-3-(4-methoxyphenyl)propan-1,3-dion (z.B. Eusolex® 9020) oder 4-Isopropyldibenzoylmethan (z.B. Eusolex® 8020),

Benzophenone wie 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40),

Methoxyzimtsäureester wie Methoxyzimtsäureoctylester (z.B. Eusolex® 2292), 4-Methoxyzimtsäureisopentylester, z.B. als Gemisch der Isomere (z.B. Neo Heliopan® E 1000),

Salicylatderivate wie 2-Ethylhexylsalicylat (z.B. Eusolex® OS), 4-Isopropylbenzylsalicylat (z.B. Megasol®) oder 3,3,5-Trimethylcyclohexylsalicylat (z.B. Eusolex® HMS),

4-Aminobenzoesäure und Derivate wie 4-Aminobenzoesäure, 4-(Dimethylamino)benzoesäure-2-ethylhexylester (z.B. Eusolex® 6007), ethoxylierter 4-Aminobenzoesäureethylester (z.B. Uvinul® P25),

Phenylbenzimidazolsulfonsäuren, wie 2-Phenylbenzimidazol-5-sulfonsäure sowie ihre Kalium-, Natrium- und Triethanolaminsalze (z.B. Eusolex® 232), 2,2-(1,4-Phenylen)-bisbenzimidazol-4,6-disulfonsäure bzw. deren Salze (z.B. Neoheliopan® AP) oder 2,2-(1,4-Phenylen)-bisbenzimidazol-6-sulfonsäure;

und weitere Substanzen wie

- 2-Cyano-3,3-diphenylacrylsäure-2-ethylhexylester (z.B. Eusolex® OCR),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX) und
- 2,4,6-Trianilino-(p-carbo-2'-ethylhexyl-1'-oxi)-1,3,5-triazin ( z.B. Uvinul® T 150) - 2-(4-Diethylamino-2-hydroxy-benzoyl)-benzoesäurehexylester (z.B. Uvinul®UVA Plus, Fa. BASF).
  Die in der Liste aufgeführten Verbindungen sind nur als Beispiele aufzufassen. Selbstverständlich können auch andere UV-Filter verwendet werden.
  Weitere geeignete organische UV-Filter sind z.B.
- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole®),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),
- $\alpha$-(Trimethylsilyl)-$\omega$-[trimethylsilyl)oxy]poly[oxy(dimethyl [und ca. 6% methyl[2-[p-[2,2-bis(ethoxycarbonyl]vinyl] phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] ($n \approx 60$) (CAS-Nr. 207 574-74-1)
- 2,2'-Methylen-bis-(6-(2H-benzotriazol-2-yl)-4-(1,1,3,3-tetramethylbutyl)phenol) (CAS-Nr. 103597-45-1)
- 2,2'-(1,4-Phenylen)bis-(1H-benzimidazol-4,6-disulfonsäure, Mononatriumsalz) (CAS-Nr. 180 898-37-7),
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6) und
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),

Weitere geeignete UV-Filter sind auch Methoxyflavone entsprechend der älteren Deutschen Patentanmeldung DE 10232595.2.

Organische UV-Filter werden in der Regel in einer Menge von 0,5 bis 20 Gewichtsprozent, vorzugsweise 1 - 15 %, in kosmetische Formulierungen eingearbeitet.

Als anorganische UV-Filter sind solche aus der Gruppe der Titandioxide wie z.B. gecoatetes Titandioxid (z.B.

Eusolex® T-2000, Eusolex®T-AQUA), Zinkoxide (z.B. Sachtotec®), Eisenoxide oder auch Ceroxide denkbar. Diese anorganischen UV-Filter werden in der Regel in einer Menge von 0,5 bis 20 Gewichtsprozent, vorzugsweise 2 - 10 %, in kosmetische Zubereitungen eingearbeitet.

Bevorzugte Verbindungen mit UV-filternden Eigenschaften sind 3-(4'-Methylbenzyliden)-dl-kampfer, 1-(4-tert-Butylphenyl)-3-(4-methoxy-phenyl)-propan-1,3-dion, 4-Isopropyldibenzoylmethan, 2-Hydroxy-4-meth-oxybenzophenon, Methoxyzimtsäureoctylester, 3,3,5-Trimethyl-cyclo-hexyl-sali-cylat, 4-(Dimethylamino)benzoesäure-2-ethyl-hexylester, 2-Cyano-3,3-di-phenylacrylsäure-2-ethylhexylester, 2-Phenyl-benzimidazol-5-sulfon-säure sowie ihre Kalium-, Natrium- und Triethanolaminsalze.

Optimierte Zusammensetzungen können beispielsweise die Kombination der organischen UV-Filter 4'-Methoxy-6-hydroxyflavon mit 1-(4-tert-Butylphenyl)-3-(4-methoxyphenyl)propan-1,3-dion und 3-(4'-Methylbenzyliden)-dl-kampfer enthalten.

Mit dieser Kombination ergibt sich ein Breitbandschutz, der durch Zusatz von anorganischen UV-Filtern, wie Titandioxid-Mikropartikeln noch ergänzt werden kann.

Alle genannten UV-Filter können auch in verkapselter Form eingesetzt werden. Insbesondere ist es von Vorteil organische UV-Filter in verkapselter Form einzusetzen. Im Einzelnen ergeben sich die folgende Vorteile:

- Die Hydrophilie der Kapselwand kann unabhängig von der Löslichkeit des UV-Filters eingestellt werden. So können beispielsweise auch hydrophobe UV-Filter in rein wässrige Zubereitungen eingearbeitet werden. Zudem wird der häufig als unangenehm empfundene ölige Eindruck beim Auftragen der hydrophobe UV-Filter enthaltenden Zubereitung unterbunden.
- Bestimmte UV-Filter, insbesondere Dibenzoylmethanderivate, zeigen in kosmetischen Zubereitungen nur eine verminderte Photostabilität. Durch Verkapselung dieser Filter oder von Verbindungen, die die Photostabilität dieser Filter beeinträchtigen, wie beispielsweise Zimtsäurederivate, kann die Photostabilität der gesamten Zubereitung erhöht werden.
- In der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.
- Allgemein können durch Verkapselung einzelner UV-Filter oder anderer Inhaltstoffe Zubereitungsprobleme, die durch Wechselwirkung einzelner Zubereitungsbestandteile untereinander entstehen, wie Kristallisationsvorgänge, Ausfällungen und Agglomeratbildung vermieden werden, da die Wechselwirkung unterbunden wird.

Daher ist es erfindungsgemäß bevorzugt, wenn ein oder mehrere der oben genannten UV-Filter in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben.

Geeignete Kapseln können Wände aus anorganischen oder organischen Polymeren aufweisen. Beispielsweise wird in US 6,242,099 B1 die Herstellung geeigneter Kapseln mit Wänden aus Chitin, Chitin-Derivaten oder polyhydroxylierten Polyaminen beschrieben. Erfindungsgemäß besonders bevorzugt einzusetzende Kapseln weisen Wände auf, die durch einen SolGel-Prozeß, wie er in den Anmeldungen WO 00/09652, WO 00/72806 und WO 00/71084 beschrieben ist, erhalten werden können. Bevorzugt sind hier wiederum Kapseln, deren Wände aus Kieselgel (Silica; undefiniertes Silicium-oxid-hydroxid) aufgebaut sind. Die Herstellung entsprechender Kapseln ist dem Fachmann beispielsweise aus den zitierten Patentanmeldungen bekannt, deren Inhalt ausdrücklich auch zum Gegenstand der vorliegenden Anmeldung gehört.

Dabei sind die Kapseln in erfindungsgemäßen Zubereitungen vorzugsweise in solchen Mengen enthalten, die gewährleisten, dass die verkapselten UV-Filter in den oben angegebenen Mengen in der Zubereitung vorliegen.

Erfindungsgemäße Bauhinia-Extrakte können in der üblichen Weise in kosmetische oder dermatologische Zubereitungen eingearbeitet werden. Geeignet sind Zubereitungen für eine äußerliche Anwendung, beispielsweise als Creme, Lotion, Gel, oder als Lösung, die auf die Haut aufgesprüht werden kann. Für eine innerliche Anwendung sind Darreichungsformeln wie Kapseln, Dragees, Pulver, Tabletten-Lösungen oder Lösungen geeignet.

Mögliche Anwendungsformen erfindungsgemäßer Zubereitungen sind beispielsweise Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Zu den bevorzugten erfindungsgemäßen Zubereitungsformen gehören insbesondere Emulsionen.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:

- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12}$-$_{15}$-Alkylbenzoat, Capryl-Caprinsäure-triglycerid, Dicaprylether.

Besonders vorteilhaft sind Mischungen aus $C_{12}$-$_{15}$-Alkylbenzoat und 2-Ethylhexylisostearat, Mischungen aus $C_{12}$-$_{15}$-Alkylbenzoat und Isotridecylisononanoat sowie Mischungen aus $C_{12}$-$_{15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann auch die Ölphase ferner einen Gehalt an cyclischen oder linearan Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate; z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemisch der vorstehend genannten Lösungsmittel verwendet. Bei alkoholischen Lösungsmitteln kann Wasser ein weiterer Bestandteil sein.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Formulierung verwendet wird.

In einer bevorzugten Ausführungsform enthalten die erfindungsgemäßen Zubereitungen hydrophile Tenside.

Die hydrophilen Tenside werden bevorzugt gewählt aus der Gruppe der Alkylglucoside, der Acyllactylate, der Betaine sowie der Cocoamphoacetate.

Die Alkylglucoside werden ihrerseits vorteilhaft gewählt aus der Gruppe der Alkylglucoside, welche sich durch die Strukturformel

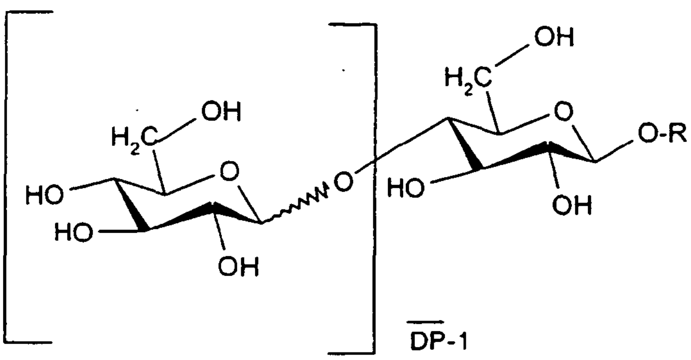

auszeichnen, wobei R einen verzweigten oder unverzweigten Alkylrest mit 4 bis 24 Kohlenstoffatomen darstellt und wobei ‚DP einen mittleren Glucosylierungsgrad von bis zu 2 bedeutet.

Der Wert ‚DP repräsentiert den Glucosidierungsgrad der erfindungsgemäß verwendeten Alkylglucoside und ist definiert als

$$\overline{DP} = \frac{p_1}{100} \cdot 1 + \frac{p_2}{100} \cdot 2 + \frac{p_3}{100} \cdot 3 + \ldots = \sum \frac{p_i}{100} \cdot i$$

Dabei stellen $p_1$, $p_2$, $p_3$ bzw. $p_i$ den Anteil der einfach, zweifach dreifach ... i-fach glucosylierten Produkte in Gewichtsprozenten dar. Erfindungsgemäß vorteilhaft werden Produkte mit Glucosylierungsgraden von 1-2, insbesondere vorteilhaft von 1, 1 bis 1,5, ganz besonders vorteilhaft von 1,2-1,4, insbesondere von 1,3 gewählt.

Der Wert DP trägt den Umstande Rechnung, dass Alkylglucoside herstellungsbedingt in der Regel Gemische aus Mono- und Oligoglucosiden darstellen. Erfindungsgemäß vorteilhaft ist ein relativ hoher Gehalt an Monoglucosiden, typischerweise in der Größenordnung von 40-70 Gew.-%.

Erfindungsgemäß besonders vorteilhaft verwendete Alkylglylcoside werden gewählt aus der Gruppe Octylglucopyranosid, Nonylglucopyranosid, Decylglucopyranosid, Undecylglucopyranosid, Dodecylglucopyranosid, Tetradecylglucopyranosid und Hexadecylglucopyranosid.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren® 1200 (Henkel KGaA), Oramix® NS 10 (Seppic).

Die Acyllactylate werden ihrerseits vorteilhaft gewählt aus der Gruppe der Substanzen, welche sich durch die Strukturformel

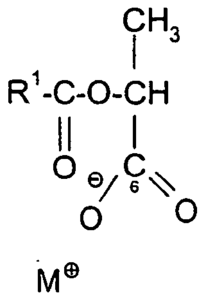

auszeichnen, wobei $R^1$ einen verzweigten oder unverzweigten Alkylrest mit 1 bis 30 Kohlenstoffatomen bedeutet und $M^+$ aus der Gruppe der Alkaliionen sowie der Gruppe der mit einer oder mehreren Alkyl- und/oder mit einer oder mehreren Hydroxyalkylresten substituierten Ammoniumionen gewählt wird bzw. dem halben Äquivalent eines Erdalkalions entspricht.

Vorteilhaft ist beispielsweise Natriumisostearyllactylat, beispielsweise das Produkt Pathionic® ISL von der Gesellschaft American Ingredients Company.

Die Betaine werden vorteilhaft gewählt aus der Gruppe der Substanzen, welche sich durch die Strukturformel

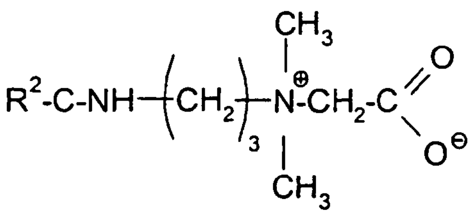

auszeichnen, wobei $R^2$ einen verzweigten oder unverzeigten Alkylrest mit 1 bis 30 Kohlenstoffatomen bedeutet.

Insbesondere vorteilhaft bedeutet $R^2$ einen verzweigten oder unverzweigten Alkylrest mit 6 bis 12 Kohlenstoffatomen.

Vorteilhaft ist beispielsweise Capramidopropylbetain, beispielsweise das Produkt Tego® Betain 810 von der Gesellschaft Th. Goldschmidt AG.

Als erfindungsgemäß vorteilhaftes Cocoamphoacetat wird beispielsweise Natriumcocoamphoacetat gewählt, wie es unter der Bezeichnung Miranol® Ultra C32 von der Gesellschaft Miranol Chemical Corp. erhältlich ist.

Die erfindungsgemäßen Zubereitungen sind vorteilhaft dadurch gekennzeichnet, dass das oder die hydrophilen Tenside in Konzentrationen von 0,01-20 Gew.-% bevorzugt 0,05-10 Gew.-%, besonders bevorzugt 0,1-5 Gew.-%, jeweils bezogen auf das Gesamtgewicht der Zusammensetzung, vorliegt oder vorliegen.

Erfindungsgemäße kosmetische und dermatologische Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Wasser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Bevorzugt werden Emulsionen. O/W-Emulsinen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Co-emulgatoren in den erfindungsgemäßen bevorzugten O/W-Emulsionen zu verwenden.

Erfindungsgemäß vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen. Insbesondere bevorzugt sind: Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17),Polyethylenglycol(18) stearylether (Steareth-18), Polyethylenglycol(19)stearylether (Steareth-19), Polyethylenglycol(20)-stearylether (Steareth-20), Polyethylenglycol(12)isostearylether (Isosteareth-12), Polyethylenglycol(13)isostearylether (Isosteareth-13), Polyethylenglycol(14)-isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol(20) isostearylether (Isosteareth-20), Polyethylenglycol(1 3)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylen-glycol(20)cetylether (Ceteth-20), Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)isocetylether (Isoceteth-17), Polyethylenglycol(18)isocetylether (Isoceteth-18), Polyethylenglycol(19)isocetylether , (Isoceteth-19), Polyethylenglycol(20)isocetylether (Isoceteth-20), Polyethylenglycol(12)oleylether (Oleth-12), Polyethylenglycol(13)oleylether (Oleth-13), Polyethylenglycol(1 4)oleylether (Oleth-14), Polyethylenglycol(1 5)oleylether (Oleth-15), Polyethylenglycol(12)laurylether (Laureth-12), Polyethylenglycol(12)-isolaurylether (Isolaureth-12), Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20).

Es ist ferner von Vorteil, die Fettsäureethoxylate ausfolgender Gruppe zu wählen:

Polyethylenglycol(20)stearat, Polyethylenglycol(21 )stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21 )isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(16)oleat, Polyethylenglycol(17)oleat,

Polyethylenglycol(18)oleat, Polyethylenglycol(19)oleat,
Polyethylenglycol(20)oleat,

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25) Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20) glyceryllaurat, Polyethylenglycol(21 )glyceryllaurat, Polyethylenglycol(22)glyceryllaurat, Polyethylenglycol(23)glyceryllaurat, Polyethylenglycol(6)glycerylcaprat/cprinat, Polyethylenglycol(20)glyceryloleat, Polyethylenglycol(20)glycerylisostearat, Polyethylenglycol(18)glyceryloleat(cocoat zu wählen.

Es ist ebenfalls günstig, die Sorbitanester aus der Gruppe

Polyethylenglycol(20)sorbitanmonolaurat,
Polyethylenglycol(20)sorbitanmonostearat,
Polyethylenglycol(20)sorbitanmonoisostearat,
Polyethyleglycol(20)sorbitanmonopalmitat,
Polyethylenglycol(20)sorbitanmonooleat zu wählen.

Als fakultative, dennoch erfindungsgemäß gegebenenfalls vorteilhafte W/O-Emulgatoren können eingesetzt werden:

Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkhole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Erfindungsgemäß bevorzugte Zubereitungen eignen sich besonders zum Schutz menschlicher Haut gegen Alterungsprozesse. Dabei liegt sie in verschiedenen, für diese Anwendung üblicherweise verwendeten Darreichungsformen vor. So kann sie insbesondere als Lotion oder Emulsion, wie als Creme oder Milch (O/W, W/O, O/W/O, W/O/W), in Form ölig-alkoholischer, ölig-wässriger oder wässrigalkoholischer Gele bzw. Lösungen, als feste Stifte vorliegen oder als Aerosol konfektioniert sein.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und außer dem erfindungsgemäßen Bauhinia-Extrakt beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen

Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die erfindungsgemäße Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfasst. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Damit erfindungsgemäße Zubereitungen ihre positive Wirkung auf die Haut besonders gut entwickeln können, kann es bevorzugt sein den erfindungsgemäßen Bauhinia-Extrakt in tiefere Hautschichten eindringen zu lassen. Dazu stehen mehrere Möglichkeiten zur Verfügung. Zum einen können der Bauhinia-Extrakt eine ausreichende Lipophilie aufweisen, um durch die äußere Hautschicht in epidermale Schichten vordringen zu können. Als weitere Möglichkeit können in der Zubereitung auch entsprechende Transportmittel, beispielsweise Liposomen, vorgesehen sein, die einen Transport der Wirkstoffe durch die äußeren Hautschichten ermöglichen.

Die erfindungsgemäßen Zubereitungen können in ebenfalls bevorzugten Ausführungsformen der Erfindung jedoch auch in der Zubereitungs-Matrix schlecht oder nicht löslichen Bauhinia-Extrakt enthalten. In diesem Fall liegen der Bauhinia-Extrakt vorzugsweise in feinteiliger Form in der kosmetischen Zubereitung dispergiert vor.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare verwendet werden. In diesem Fall erfolgt geeignet eine Konfektionierung als Shampoo, Lotion, Gel oder Emulsion zum Ausspülen, wobei die jeweilige Zubereitung vor oder nach dem Shamponieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Eine erfindungsgemäße Zubereitung mit Lichtschutzeigenschaften kann außer dem Bauhinia-Extrakt verschiedene, in diesem Mitteltyp verwendete Adjuvantien enthalten, wie grenzflächenaktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Die erfindungsgemäßen Zubereitungen können durch ein Verfahren hergestellt werden **dadurch gekennzeichnet, dass** ein wässriger oder hydroalkoholischer Extrakt aus Bauhinia sowie mindestens ein weiterer hautpflegender Inhaltsstoff ausgewählt unter Pyrimidincarbonsäuren und/oder Aryloximen, vorzugsweise Ectoin mit mindestens einem für topische Anwendungen geeigneten Träger, bevorzugt mit einem kosmetisch oder dermatologisch geeigneten Träger vermischt wird. Das Vermischen kann ein Lösen, Emulgieren oder Dispergieren des Bauhinia-Extrakts in dem Träger zur Folge haben.

Die erfindungsgemäßen Zubereitungen können dabei mit Hilfe von Techniken hergestellt werden, die dem Fachmann wohl bekannt sind.

Im folgenden wird die Erfindung anhand von Beispielen näher erläutert. die Erfindung ist im gesamten beanspruchten Bereich ausführbar und nicht auf die hier genannten Beispiele beschränkt.

**<u>Beispiel 1:</u> Herstellung eines Trockenextrakts aus Bauhinia forficata mithilfe hydroalkoholischer Extraktion**

**1. Hydroalkoholische Extraktion mit Ethanol 50 (v/v)**

1 kg trockene, verriebene Blätter von Bauhinia fortificata und 6 L einer hydroalkoholischen Lösung 50% (v/v) werden in ein Glasreaktionsgefäß gegeben und unter Rückfluss bei kontinuierlichem Rühren etwa eine Stunde erhitzt. Unter Verwendung eines Büchner Porzellan-Filters mit einem Filterpapier wird der Extrakt filtriert.

In einem zweiten Extraktionsschritt wird dasselbe Volumen hydroalkoholischer Lösung 50% (v/v) zum verbleibenden Volumen in das Glasreaktionsgefäß gegeben, unter Rückfluss bei kontinuierlichem Rühren etwa eine Stunde erhitzt und unter Verwendung eines Büchner Porzellan-Filters mit einem Filterpapier filtriert.

**2. Vorkonzentrierung**

Der zuvor erhaltene Extrakt wird bis zu einem Gesamttrockenanteil 5,0% and 6,0% in einem Glas Evaporator/ Konzentrator unter Vakuum (500 mmHg) konzentriert.

### 3. Spray-Trocknung

Das erhaltene Konzentrat wird in einem Mini Spray Dryer B-191 (Büchi) unter folgenden Bedingungen spraygetrocknet:

Einlasstemperatur: 120 °C
Auslasstemperatur: 65-70 °C
Pumpe: 15%
Aspirator: 90%
Luftzufluss: 400 ml/min
Pumpenfluss: ~220 ml/h
Reiningungstemporizer: 2
Liniendruck: 80 psi (5,5 bar)

### 4. Analytik

Die Extrakte werden mithilfe von Hochdruckflüssigkeitschromatographie (HPLC) und Dünnschicht-Chromatographie (TLC) charakterisiert.

### Beispiel 2: antioxidatives Potential des hydroalkoholischen Bauhinia-Extrakts

Das antioxidative Potential des hydroalkoholischen Extrakts aus Beispiel 1 wird als Trolox Equivalent Antioxidans-Wirkung (TEAC-Assay), EC50 (DPPH-Assay) und relative antioxidante Effizienz (RAE; Lipid-Assay) nach Halliwell, B.; Eschbach, R.; Löhliger, J.; Aruoma, O.I.; Food. Chem. Toxicol. Vol 33, p. 601-67, 1995 (table 8) bestimmt.

Tabelle 1: antioxidatives Potential des Extrakts aus Beispiel 1

| Verfahren | Ergebnis |
|---|---|
| TEAC-assay | TEAC = 0.12 (bezogen auf tmg/l) |
| DPPH-assay | EC50 = 0.37 (bez. auf mg/l bis μmol/l) |
| Lipid-assay | RAE = 0.19 |

### Beispiel 3: Wirkung eines hydroalkoholischen Bauhinia-Extrakts

Zur Stimulierung der Kollagen-Produktion werden menschliche Fibroblasten mit Vitamin C kultiviert. Die Zellen werden dann durch serielles Einfrieren/Auftauen lysiert und mit D-[5-$^3$H]-Glucose über 15 Tage markiert. Am Ende der Inkubation werden die Proteine extrahiert und präzipitiert. Dann wird das Pellet gewaschen und am Ende des Experiments wird die kovalent an die Fasern gebundene Glucose durch Flüssigkeitsscintilation gemessen.
Der hydroalkoholische Bauhinia-Extrakt (s. Beispiel 1) zeigt eine signifikante Verminderung der Protein-Glykosylierung (55% der Kontrolle) bei einer Konzentration von 0.03 mg/ml.

### Beispiel 4: Herstellung eines Trockenextrakts aus Bauhinia forficata mithilfe wässriger Extraktion

Die Herstellung eines Trockenextrakts aus Bauhinia forficata durch wässrige Extraktion erfolgt gemäß obengenannten Verfahren zur Herstellung eines hydroalkoholischen Extrakts, wobei allerdings anstelle eines polaren Gemischs (hydroalkoholisches Gemisch) Wasser als Extraktionsmittel verwendet wird.

### Beispiel 5: Wirkung eines wässrigen Bauhinia-Extrakts

**Material:**

- menschliche, dermale Fibroblasten (NDHF Pool PF2)
- Kulturmedium: DMEM,2 mM Glutamin, 50 UI/ml Penicillin, 50 μg/ml Streptomycin, 10 % fötales Kälberserum (Life Technologies)

- Trockenextrakt aus Bauhinia forficata nach Beispiel 4 (wässrige Extraktion), gelöst in sterilem PBS
- getestete Endkonzentrationen: 0,3 mg/ml, 0,1 mg/ml, 0,03 mg/ml
- Referenz: Aminoguanidin 1 mg/ml

**Methodik:**

Menschliche dermale Fibroblasten werden in Zellkulturmedium (s.o.) in $25cm^2$-Platten bei 37°C und 5% $CO_2$ zur Konfluenz kultiviert. Durch Kultivierung der Zellen in Gegenwart von Vitamin C (Endkonzentration: 20 µ/ml) über 96 h wird die Kollagen-Synthese stimuliert. Die Zellen werden mit PBS gewaschen und durch serielles Einfrieren/Auftauen lysiert. Die Zellen werden mit wässrigem Extrakt aus Bauhinia forficata oder Aminoguanidin (und Kontrolle: PBS) behandelt und mit 20µCi/ml D-[5-$^3$H]-Glucose (19 Ci/mmol), 703 Gbq/mmol) in Gegenwart von 0,1 mg/ml Glucose markiert. Die Behandlung und Markierung erfolgt ohne Unterbrechung über 15 Tage bei 37°C und 5% $CO_2$.
Am Ende der Inkubation werden Proteine der extrazellulären Matrix (hauptsächlich Kollagen) durch Puffer (Tris 50 mM, 4 M Guanidin, 5 mM EDTA, pH 8,0, 30 min) extrahiert. Das Gesamtprotein wird durch 20% (v/v) Trichloressigsäure, 0,66 mg/ml Albumin ausgefällt. Die Präzipitate werden auf Filtern unter Verwendung eines Skatron-Kollektors wiedergewonnen und ausgiebig gewaschen, zuerst in 3% Trichloressigsäure, dann mit 70% Ethanol. Schließlich wird die kovalent an die Fasern der extrazellulären Matrix gebundene Glukose durch Scintillation gemessen.

**Ergebnisse und Diskussion:** (siehe auch Tabelle 1 und Abbildung 1)

Der basale nicht-enzymatische Einbau von Glukose in Proteine (größer als 20.000 cpm/Well) liefert einen adäquaten Vergleichswert für die Untersuchung von Produkten, die die Proteinglycation beeinflussen. Aminoguanidin (1 mg/ml) als Referenzwert vermindert den Glucose-Einbau um 85%. Dieser Wert validiert den Assay. Auch der wässrige Extrakt aus Bauhinia forficata (0,3 mg/ml, 0,1 mg/ml bzw. 0,03 mg/ml) vermindert den nicht-enzymatischen Einbau von Glukose in Proteine signifikant (58%, 77% bzw. 66% der Kontrolle, $p < 0.01$). Folglich vermag ein wässriger Extrakt aus Bauhinia forficata in den getesteten Konzentrationen (0,3 mg/ml, 0,1 mg/ml bzw. 0,03 mg/ml) die Glycation von Proteinen der extrazellulären Matrix in-vitro zu vermindern.

**Tabelle 2:**

| Wirkung der Behandlung mit einem wässrigen Extrakt aus Bauhinia forficata auf den nicht-enzymatischen Einbau von Glukose in Proteine (Glycation) in Fibroblasten | | | | | | |
|---|---|---|---|---|---|---|
| **Behandlung** | **cpm** | **Mittelwert** | **Standard-abweichung** | **n** | **% der Kontrolle** | **p** |
| **Kontrolle** | 27267<br>23736<br>21282<br>25147<br>23290<br>26823 | **24591** | 2273 | 6 | **100** | - |
| **Aminoguanidin 1 mg/ml** | 2859<br>3434<br>4809 | **3701** | 1002 | 3 | **15** | *p < 0.01* |
| **wässriger Extrakt aus Bauhinia forficata 0.3 mg/ml** | 13101<br>14384<br>15063 | **14183** | 996 | 3 | **58** | *p < 0.01* |
| **wässriger Extrakt aus Bauhinia forficata 0.1 mg/ml** | 19807<br>18771 -<br>17859 | **18812** | 975 | 3 | **77** | *p < 0.01* |

(fortgesetzt)

| Wirkung der Behandlung mit einem wässrigen Extrakt aus Bauhinia forficata auf den nicht-enzymatischen Einbau von Glukose in Proteine (Glycation) in Fibroblasten | | | | | | |
|---|---|---|---|---|---|---|
| **Behandlung** | **cpm** | **Mittelwert** | **Standard-abweichung** | **n** | **% der Kontrolle** | **p** |
| **wässriger Extrakt aus Bauhinia forficata 0.03 mg/ml** | 17463<br>15459<br>15845 | **16256** | 1063 | 3 | **66** | *$p < 0.01$* |

**Patentansprüche**

1. Zubereitung enthaltend einen wässrigen oder hydroalkoholischen Extrakt aus Bauhinia sowie mindestens einen weiteren hautpflegenden Inhaltsstoff ausgewählt unter Pyrimidincarbonsäuren und/oder Aryloximen, vorzugsweise Ectoin und mindestens einen für topische Anwendungen geeigneten Träger.

2. Zubereitung nach Anspruch 1, enthaltend mindestens einen weiteren Wirkstoff, ausgewählt unter Tilirosid, Aspirin, Lysin, Arginin, Carnosin, Ethanolamin, Aminoguanidin, Pyridoxamin, Thiaminpyrophosphat, Kinetin, Vitamin C und Vitamin E.

3. Zubereitung nach einem oder mehreren der vorstehenden Ansprüche, enthaltend einen oder mehrere UV-Filter, ausgewählt unter 3-(4'-Methylbenzyliden)-dl-kampfer, 1-(4-tert-Butylphenyl)-3-(4-methoxyphenyl)propan-1,3-dion, 4-Isopropyldibenzoylmethan, 2-Hydroxy-4-methoxybenzophenon, Methoxyzimtsäureoctylester, 3,3,5-Trimethylcyclohexylsalicylat, 4-(Dimethylamino)benzoesäure-2-ethylhexylester, 2-Cyano-3,3-diphenylacrylsäure-2-ethylhexylester und 2-Phenylbenzimidazol-5-sulfonsäure sowie ihren Kalium-, Natrium- und Triethanolaminsalzen, anorganischen Filtern und verkapselten Filtern.

4. Zubereitung nach einem oder mehreren der vorstehenden Ansprüche, **dadurch gekennzeichnet, dass** die Zubereitung einen wässrigen oder hydroalkoholischen Extrakt aus einer Bauhinia-Species enthält, ausgewählt aus Bauhinia candicans, Bauhinia championii, Bauhinia fortificata (= Bauhinia forficata), Bauhinia manca, Bauhinia purpurea, Bauhinia racemosa, Bauhinia reticulata, Bauhinia tomentosa, Bauhinia variegata, Bauhinia cheilantha, Bauhinia guanensis, Bauhinia refusa, Bauhinia glauca, Bauhinia pauletia, Bauhinia ungulato, Bauhinia macrostachya und Bauhinia splendens.

5. Zubereitung nach einem oder mehreren der vorstehenden Ansprüche, **dadurch gekennzeichnet, dass** die Zubereitung einen wässrigen oder hydroalkoholischen Extrakt aus Bauhinia in einer Menge von 0,001 bis 20 Gew.-%, vorzugsweise in einer Menge von 0,01 bis 10 Gew.-% enthält.

6. Zubereitung nach einem oder mehreren der vorstehenden Ansprüche, **dadurch gekennzeichnet, dass** der wässrige Bauhinia-Extrakt erhalten wird durch wässrige Extraktion aus Teilen von Bauhinia.

7. Zubereitung nach einem oder mehreren der Ansprüche 1 bis 5, **dadurch gekennzeichnet, dass** der hydroalkoholische Bauhinia-Extrakt erhalten wird durch

   a) Extraktion aus Teilen von Bauhinia mithilfe eines Gemischs polarer Lösungsmittel und
   b) anschließendes Verwerfen des Lösungsmittels.

8. Zubereitung nach Anspruch 7, **dadurch gekennzeichnet, dass** das Gemisch polarer Lösungsmittel Wasser als ein Lösungsmittel und mindestens ein weiteres Lösungsmittel umfasst, ausgewählt aus Methanol, Ethanol, 1-Propanol, 2-Propanol, Acetonitril, Aceton und Ethylacetat und ein bevorzugtes Gemisch 10 - 90% (Vol) Wasser und besonders bevorzugt 30 - 70% (Vol) Wasser enthält.

## Claims

1. Composition comprising an aqueous or hydroalcoholic extract from bauhinia and at least one further skin-care ingredient selected from pyrimidinecarboxylic acids and/or aryl oximes, preferably ectoin, and at least one vehicle which is suitable for topical applications.

2. Composition according to Claim 1, comprising at least one further active ingredient selected from tiliroside, aspirin, lysine, arginine, carnosine, ethanolamine, aminoguanidine, pyridoxamine, thiamine pyrophosphate, kinetin, vitamin C and vitamin E.

3. Composition according to one or more of the preceding claims, comprising one or more UV filters selected from 3-(4'-methylbenzylidene)-dl-camphor, 1-(4-tert-butylphenyl)-3-(4-methoxyphenyl)propane-1,3-dione, 4-isopropyldibenzoyl-methane, 2-hydroxy-4-methoxybenzophenone, octyl methoxycinnamate, 3,3,5-trimethylcyclohexyl salicylate, 2-ethylhexyl 4-(dimethylamino)benzoate, 2-ethylhexyl 2-cyano-3,3-diphenylacrylate and 2-phenylbenzimidazole-5-sulfonic acid and potassium, sodium and triethanolamine salts thereof, inorganic filters and encapsulated filters.

4. Composition according to one or more of the preceding claims, **characterised in that** the composition comprises an aqueous or hydroalcoholic extract from a bauhinia species selected from Bauhinia candicans, Bauhinia championii, Bauhinia fortificata (= Bauhinia forficata), Bauhinia manca, Bauhinia purpurea, Bauhinia racemosa, Bauhinia reticulata, Bauhinia tomentosa, Bauhinia variegata, Bauhinia cheilantha, Bauhinia guanensis, Bauhinia refusa, Bauhinia glauca, Bauhinia pauletia, Bauhinia ungulato, Bauhinia macrostachya and Bauhinia splendens.

5. Composition according to one or more of the preceding claims, **characterised in that** the composition comprises an aqueous or hydroalcoholic extract from bauhinia in an amount of from 0.001 to 20% by weight, preferably in an amount of from 0.01 to 10% by weight.

6. Composition according to one or more of the preceding claims, **characterised in that** the aqueous bauhinia extract is obtained by aqueous extraction from parts of bauhinia.

7. Composition according to one or more of Claims 1 to 5, **characterised in that** the hydroalcoholic bauhinia extract is obtained by

   a) extraction from parts of bauhinia with the aid of a mixture of polar solvents and
   b) subsequent discarding of the solvent.

8. Composition according to Claim 7, **characterised in that** the mixture of polar solvents includes water as one solvent and at least one further solvent selected from methanol, ethanol, 1-propanol, 2-propanol, acetonitrile, acetone and ethyl acetate, and a preferred mixture comprises 10 - 90% (vol.) of water and particularly preferably 30 - 70% (vol.) of water.

## Revendications

1. Composition comprenant un extrait aqueux ou hydroalcoolique de bauhinia et au moins un autre ingrédient de soin pour la peau choisi parmi les acides pyrimidine carboxyliques et/ou les aryloximes, préférablement l'ectoïne, et au moins un véhicule convenable pour des applications topiques.

2. Composition selon la revendication 1, comprenant au moins un autre ingrédient actif choisi parmi la tiliroside, l'aspirine, la lysine, l'arginine, la carnosine, l'éthanolamine, l'aminoguanidine, la pyridoxamine, le pyrophosphate de thiamine, la kinétine, la vitamine C et la vitamine E.

3. Composition selon l'une ou plusieurs des revendications précédentes, comprenant un ou plusieurs filtres UV choisis parmi le 3-(4'-méthylbenzylidène)-dl-camphre, la 1-(4-tertio-butylphényl)-3-(4-méthoxyphényl)propane-1,3-dione, le 4-isopropyldibenzoylméthane, la 2-hydroxy-4-méthoxybenzophénone, le méthoxycinnamate d'octyle, le salicylate de 3,3,5-triméthylcyclohexyle, le 4-(diméthylamino)benzoate de 2-éthylhexyle, le 2-cyano-3,3-diphénylacrylate de 2-éthylhexyle et l'acide 2-phénylbenzimidazole-5-sulfonique et les sels de potassium, de sodium et de triéthanolamine de ceux-ci, les filtres inorganiques et les filtres encapsulés.

4. Composition selon l'une ou plusieurs des revendications précédentes, **caractérisée en ce que** la composition comprend un extrait aqueux ou hydroalcoolique d'une espèce de bauhinia choisie parmi Bauhinia candicans, Bauhinia championii, Bauhinia fortificata (= Bauhinia forficata), Bauhinia manca, Bauhinia purpurea, Bauhinia racemosa, Bauhinia reticulata, Bauhinia tomentosa, Bauhinia variegata, Bauhinia cheilantha, Bauhinia guanensis, Bauhinia refusa, Bauhinia glauca, Bauhinia pauletia, Bauhinia ungulato, Bauhinia macrostachya et Bauhinia splendens.

5. Composition selon l'une ou plusieurs des revendications précédentes, **caractérisée en ce que** la composition comprend un extrait aqueux ou hydroalcoolique de bauhinia en une quantité allant de 0,001 à 20% en poids, préférablement en une quantité allant de 0,01 à 10% en poids.

6. Composition selon l'une ou plusieurs des revendications précédentes, **caractérisée en ce que** l'extrait aqueux de bauhinia est obtenu par extraction aqueuse de parties de bauhinia.

7. Composition selon l'une ou plusieurs des revendications 1 à 5, **caractérisée en ce que** l'extrait hydroalcoolique de bauhinia est obtenu par

a) extraction de parties de bauhinia à l'aide d'un mélange de solvants polaires et
b) élimination ultérieure du solvant.

8. Composition selon la revendication 7, **caractérisée en ce que** le mélange de solvants polaires comporte de l'eau comme l'un des solvants et au moins un autre solvant choisi parmi le méthanol, l'éthanol, le 1-propanol, le 2-propanol, l'acétonitrile, l'acétone et l'acétate d'éthyle, et un mélange préféré comprend 10

- 90% (vol.) d'eau et particulièrement préférablement 30 - 70% (vol.) d'eau.

**Abbildung 1:** Wirkung der Behandlung mit einem wässrigen Extrakt aus Bauhinia forficata auf den nicht-enzymatischen Einbau von Glukose in Proteine (Glycation) in Fibroblasten

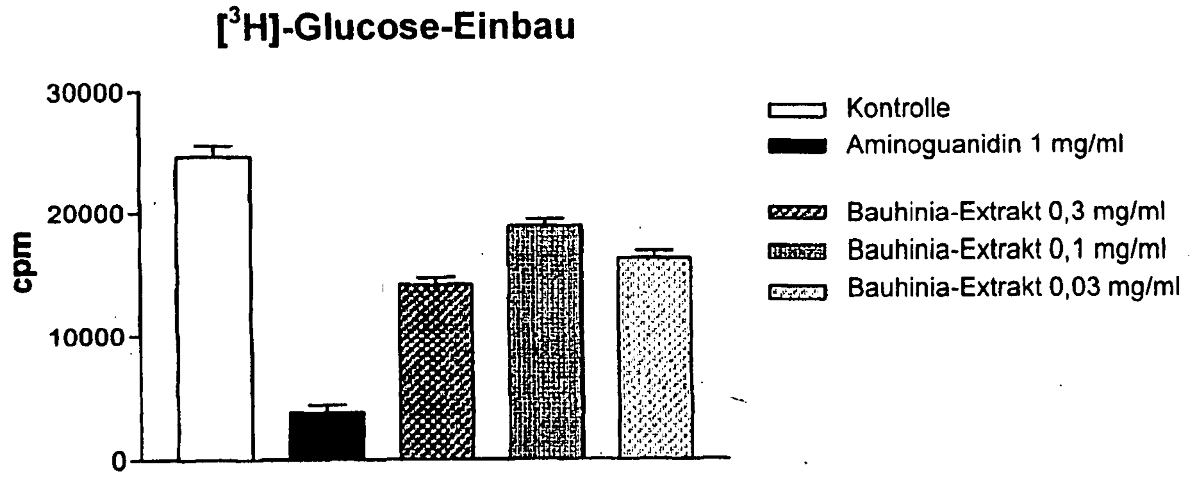

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9319743 A
- WO 0202074 A
- EP 0671161 A
- DE 4116123 A
- DE 10232595
- US 6242099 B1
- WO 0009652 A
- WO 0072806 A
- WO 0071084 A
- DE 4308282 A


### In der Beschreibung aufgeführte Nicht-Patentliteratur


- **Campisi J.** The role of cellular senescence in skin aging. *J. Investig. Dermatol. Symp. Proc.,* 1998, vol. 3, 1-5
- **Biesalski H. K.** Free radical theory of aging. *Curr. Opin. Clin. Nutr. Metab. Care,* 2002, vol. 5
- **Kasper, M. ; Funk, R. H. W.** Age-related changes in cells and tissues due to advanced glycation end products (AGEs). *Archives of Gerontology and Geriatrics,* 2001, vol. 32, 233-243
- **Reiser, K. M.** Nonenzymatic glycation of collagen in aging & diabetes. *Proc. Soc. Exp. Biol. Med.,* 1998, vol. 218, 23-37
- **Khalifah, R. G. et al.** Amadorins: novel post-Amadori inhibitors of advanced glycation reactions. *Biochem. Biophys. Res. Commun.,* 1999, vol. 257, 251-258
- **Verbeke P. et al.** Kinetin inhibits protein oxidation and glycoxidation in vitro. *Biochem. Biophys. Res. Commun.,* 2000, vol. 276, 1265-1270
- **Cerami A.** Hypothesis: Glucose as a Mediator of Aging. *Journal of the American Geriatric Society,* vol. 33 (9), 626-634
- **Hayase F. et al.** Aging of proteins: immunological detection of a glucose-derived pyrrole formed during maillard reaction in vivo. *J. Biol. Chem.,* 05. Marz 1989, vol. 264 (7), 3758-64
- **Verzijl N. et al.** Age-related accumulation of Maillard reaction products in human articular cartilage collagen. *Biochem. J.,* 01. September 2000, vol. 350 (2), 381-7
- **Beisswenger, P. J. et al.** *Diabetes Care,* 1993, vol. 16, 689-694
- **K. Lemanska ; H. Szymusiak ; B. Tyrakowska ; R. Zielinski ; I.M.C.M. Rietjens.** *Current Topics in Biophysics,* 2000, vol. 24 (2), 101-108
- **C.A. Rice-Evans ; N.J. Miller ; G. Paganga.** *Trends in Plant Science,* 1997, vol. 2 (4), 152-159
- **K. Lemanska ; H. Szymusiak ; B. Tyrakowska ; R. Zielinski ; A.E.M.F. Soffers ; I.M.C.M. Rietjens.** *Free Radical Biology&Medicine,* 2001, vol. 31 (7), 869-881
- **E.A. Galinski et al.** *Eur. J. Biochem.,* 1985, vol. 149, 135-139
- **Halliwell, B. ; Eschbach, R. ; Löhliger, J. ; Aruoma, O.I.** *Food. Chem. Toxicol.,* 1995, vol. 33, 601-67